# EUROPEAN PATENT APPLICATION

(11) **EP 4 737 544 A1**
(43) Date of publication of application: **06.05.2026**
(21) Application number: 24831734.9
(22) Date of filing: 17.06.2024
(51) Int. Cl.: C11B 9/00, A61K 8/40, A61Q 13/00, C07C 253/30, C07C 255/07

(54) **PERFUME COMPOSITION**

(30) Priority: 29.06.2023 JP 2023107515
(71) Applicant: MITSUBISHI GAS CHEMICAL COMPANY, INC., Chiyoda-ku Tokyo 100-8324 (JP)
(72) Inventor: UTAMURA, Tatsuya, Kurashiki-shi, Okayama 712-8525 (JP); NISHIUCHI, Junya, Kurashiki-shi, Okayama 712-8525 (JP)
(74) Representative: Müller-Boré & Partner Patentanwälte PartG mbB
(86) International application number: PCT/JP2024/021791
(87) International publication number: WO 2025/004871

(57) **Abstract**

Provided is a fragrance composition containing the following 3-(4-alkylphenyl)propanenitrile represented by General Formula (1) as an active ingredient. The fragrance composition contains 3-(4-alkylphenyl)propanenitrile having a floral scent, and a green scent or a fruity scent, as an active ingredient and has a strong floral scent. Further, use of 3-(4-alkylphenyl)propanenitrile as a fragrance, a scenting method, and a method for producing 3-(4-alkylphenyl)propanenitrile are provided. In Formula (1), R represents an alkyl group having from 2 to 4 carbon atoms.

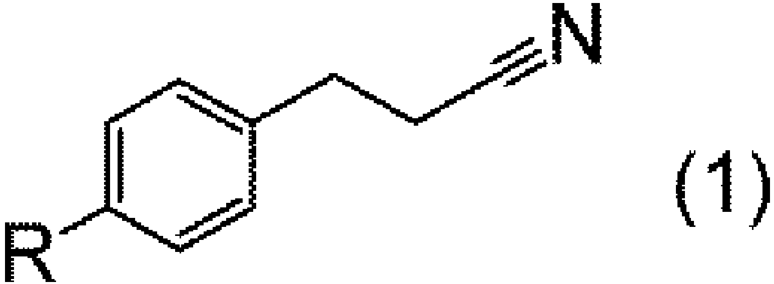

## Description

### Technical Field

The present invention relates to a fragrance composition.

### Background Art

As nitrile compounds having a phenyl group, there are known, as fragrance components, Peonile (2-cyclohexylidene-2-phenylacetonitrile) having a grapefruit, geranium, rose floral-like aroma, cinnamyl nitrile having a cinnamon, cassia-like aroma, and the like (Non-Patent Literature 1).

It is known that some nitriles corresponding to aldehydes used as fragrances have similar aromatic notes.

Non-Patent Literature 2 describes a method for deriving a corresponding nitrile from aldehydes used as a fragrance, and that the derived nitrile is useful as a fragrance component.

### Citation List

### Non-Patent Literature

Non-Patent Literature 1: "Zoho Shin pan, Gousei Koryo: Kagaku to Shohin Chisiki (Enlarged and Revised Edition, Synthetic Fragrance: Chemistry and Product Knowledge)" edited by the Synthetic Fragrance Editing Committee, The Chemical Daily Co. Ltd., 2016, pages 725 to 726
Non-Patent Literature 2: Flavor Fragrance Journal, 2020, vol. 35, pp. 425 to 435

### Summary of Invention

### Technical Problem

As described above, although some nitrile compounds have been used as fragrances, a novel scent is required to increase value of products, such as fragrance products, cosmetic products, detergents, sanitary products, general goods, medical and pharmaceutical products, and food products. Typically, a fragrance is used as a blended fragrance obtained by mixing a plurality of fragrances, and a fragrance component exhibiting an excellent effect especially when mixed with another fragrance is demanded.

An object of the present invention is to provide a fragrance composition having novel scent and characteristics, use of a compound that can impart novel scent and characteristics to the fragrance composition, and a scenting method.

### Solution to Problem

The present inventors have found that a fragrance composition contains a specific nitrile compound as an active ingredient and therefore has novel scent and characteristics, that the nitrile compound can be used as a fragrance, and that the nitrile compound can impart a scent useful as a fragrance, and thus completed the present invention.

That is, the present invention is as follows.
[1] A fragrance composition, containing 3-(4-alkylphenyl)propanenitrile represented by General Formula (1) as an active ingredient: 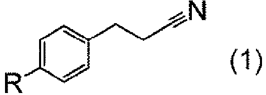 where in Formula (1), R represents an alkyl group having from 2 to 4 carbon atoms.
[2] The fragrance composition according to [1], further containing a fragrance substance other than the 3-(4-alkylphenyl)propanenitrile represented by Formula (1).
[3] The fragrance composition according to [2], wherein the fragrance substance other than the 3-(4-alkylphenyl)propanenitrile represented by Formula (1) contains at least one selected from the group consisting of hydrocarbons, alcohols, esters, phenols, aldehydes, ketones, acetals, ketals, ethers, natural essential oils, natural extracts, and nitriles other than the 3-(4-alkylphenyl)propanenitrile represented by Formula (1).
[4] The fragrance composition according to any one of [1] to [3], wherein R is at least one selected from the group consisting of an ethyl group, an isopropyl group, an n-butyl group, an isobutyl group, and a tert-butyl group.
[5] The fragrance composition according to any one of [1] to [4], wherein R is at least one selected from the group consisting of an isopropyl group, an n-butyl group, and an isobutyl group.
[6] The fragrance composition according to any one of [1] to [5], wherein R is an n-butyl group.
[7] The fragrance composition according to any one of [1] to [6], wherein 3-(2-alkylphenyl)propanenitrile represented by General Formula (2) is contained in an amount of from 0.03 to 0.8 mass% based on the 3-(4-alkylphenyl)propanenitrile represented by Formula (1): 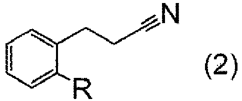 where in Formula (2), R represents an alkyl group having from 2 to 4 carbon atoms.
[8] Use of 3-(4-alkylphenyl)propanenitrile represented by General Formula (1) as a fragrance: 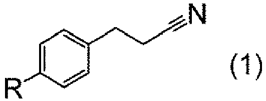 where in Formula (1), R represents an alkyl group having from 2 to 4 carbon atoms.
[9] The use according to [8], wherein R is at least one selected from the group consisting of an ethyl group, an isopropyl group, an n-butyl group, an isobutyl group, and a tert-butyl group.
[10] The use according to [8] or [9], wherein the 3-(4-alkylphenyl)propanenitrile represented by Formula (1) imparts a floral scent, and a green scent or a fruity scent.
[11] The use according to any one of [8] to [10], wherein R is at least one selected from the group consisting of an isopropyl group and an isobutyl group.
[12] The use according to [11], wherein the 3-(4-alkylphenyl)propanenitrile represented by Formula (1) imparts a floral scent, a green scent, and a fruity scent.
[13] The use according to any one of [8] to [10], wherein R is an n-butyl group.
[14] The use according to [13], wherein the 3-(4-alkylphenyl)propanenitrile represented by Formula (1) imparts a floral scent, a fruity scent, and a spicy scent.
[15] A scenting method including imparting a floral scent, and a green scent or a fruity scent, with 3-(4-alkylphenyl)propanenitrile represented by General Formula (1): 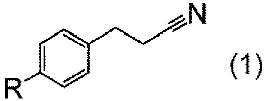 where in Formula (1), R represents an alkyl group having from 2 to 4 carbon atoms.
[16] A method for producing 3-(4-alkylphenyl)propanenitrile, the method including, in this order: a step of converting, via a condensation reaction, 4-alkylbenzaldehyde represented by Formula (3) as a raw material and acetonitrile into cinnamonitrile represented by Formula (4); and a hydrogenation step: 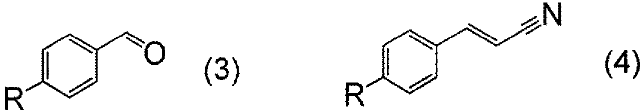 where in Formulae (3) and (4), R represents an alkyl group having from 2 to 4 carbon atoms.
[17] A nitrile composition containing 3-(4-alkylphenyl)propanenitrile represented by General Formula (1) and 3-(2-alkylphenyl)propanenitrile represented by General Formula (2), wherein the 3-(2-alkylphenyl)propanenitrile represented by General Formula (2) is contained in an amount of from 0.03 to 0.8 mass% based on the 3-(4-alkylphenyl)propanenitrile represented by Formula (1): 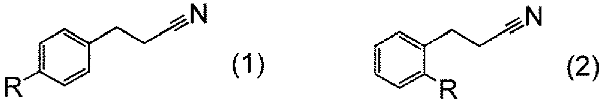 where in Formula (1), R represents an alkyl group having from 2 to 4 carbon atoms, and, in Formula (2), R represents an alkyl group having from 2 to 4 carbon atoms.

### Advantageous Effects of Invention

The present invention can provide a fragrance composition containing 3-(4-alkylphenyl)propanenitrile capable of imparting a floral scent, and a green scent or a fruity scent, as an active ingredient and therefore having a strong floral scent. Additionally, provided are use of 3-(4-alkylphenyl)propanenitrile having a floral scent, and a green scent or a fruity scent, as a fragrance, a scenting method, and a method for producing 3-(4-alkylphenyl)propanenitrile.

### Description of Embodiments

Hereinafter, in the present description, the expression "from XX to YY" means "XX or more and YY or less", "XX or higher and YY or lower", or "XX or greater and YY or less".

### [Fragrance composition]

The fragrance composition of the present invention is a fragrance composition containing 3-(4-alkylphenyl)propanenitrile represented by General Formula (1) set forth below as an active ingredient: 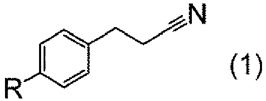 where in Formula (1), R represents an alkyl group having from 2 to 4 carbon atoms.

The fragrance composition of the present invention contains 3-(4-alkylphenyl)propanenitrile having a floral scent, and a green scent or a fruity scent, as an active ingredient, and therefore has a strong floral scent and is excellent in scent intensity. Thus, it is useful as an aromatic component of various products.

### <3-(4-alkylphenyl)propanenitrile>

In Formula (1), R represents an alkyl group having from 2 to 4 carbon atoms. Specifically, R is preferably at least one selected from the group consisting of an ethyl group, an isopropyl group, an n-butyl group, an isobutyl group, and a tert-butyl group, more preferably at least one selected from the group consisting of an isopropyl group, an n-butyl group, and an isobutyl group, and even more preferably an n-butyl group.

When R is at least one selected from the group consisting of an ethyl group, an isopropyl group, an n-butyl group, an isobutyl group, and a tert-butyl group, the compound has a green scent or a fruity scent in addition to a floral scent, and can impart a strong floral scent, and therefore is very useful as an active ingredient of the fragrance composition.

When R is at least one selected from the group consisting of an isopropyl group and an isobutyl group, the compound has a green scent and a fruity scent in addition to a floral scent, and can impart a strong floral scent, and therefore is very useful as an active ingredient of the fragrance composition.

When R is an n-butyl group, the compound has a fruity scent and a spicy scent in addition to a floral scent, and can impart a strong floral scent, and therefore is very useful as an active ingredient of the fragrance composition. In addition, when R is an n-butyl group, the intensity and diffusibility of the scent of the fragrance composition can be further significantly improved.

The 3-(4-alkylphenyl)propanenitrile represented by Formula (1) is useful as an active ingredient of the fragrance composition, has a floral scent, and a green scent or a fruity scent, can impart a strong floral scent, and can further impart the intensity and diffusibility of the scent.

When R is an ethyl group, the compound especially has a green scent in addition to a floral scent, and imparts a strong floral scent.

When R is an isopropyl group, the compound especially has a green scent and a fruity scent in addition to a floral scent, and imparts a strong floral scent.

When R is an n-butyl group, the compound especially has a fruity scent and a spicy scent in addition to a floral scent, and imparts a strong floral scent. Furthermore, the intensity and diffusibility of the scent of the fragrance composition can be remarkably improved.

When R is an isobutyl group, the compound especially has a green scent and a fruity scent in addition to a floral scent, and imparts a strong floral scent.

When R is a tert-butyl group, the compound especially has a green scent in addition to a floral scent, and imparts a strong floral scent.

The fragrance composition of the present invention preferably contains 3-(2-alkylphenyl)propanenitrile represented by General Formula (2) set forth below.

The fragrance composition of the present invention more preferably contains from 0.03 to 0.8 mass% of 3-(2-alkylphenyl)propanenitrile represented by Formula (2) based on the 3-(4-alkylphenyl)propanenitrile represented by Formula (1).

It is considered that a more complex and better aromatic note is obtained due to the 3-(2-alkylphenyl)propanenitrile represented by Formula (2) being contained in a small amount: 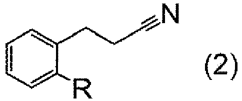 where in Formula (2), R represents an alkyl group having from 2 to 4 carbon atoms.

R in Formula (2) is an alkyl group having from 2 to 4 carbon atoms, and is preferably the same as the 3-(4-alkylphenyl)propanenitrile of Formula (1) contained in the fragrance composition. That is, when 3-(4-n-butylphenyl)propanenitrile is contained as an active ingredient in the fragrance composition, 3-(2-n-butylphenyl)propanenitrile is preferably contained.

A content of the 3-(4-alkylphenyl)propanenitrile in the fragrance composition of the present invention may be appropriately adjusted according to the type of fragrance composition, the type of target aroma, the intensity of the aroma, and the like and is preferably from 0.01 to 90 mass%, more preferably from 0.1 to 50 mass%, even more preferably from 0.5 to 30 mass%, yet even more preferably from 1.0 to 20 mass%, and yet even more preferably from 1.5 to 10 mass%.

### <Additional component and product that can be used>

Examples of an additional component that is contained in the fragrance composition containing the 3-(4-alkylphenyl)propanenitrile and that is other than the 3-(4-alkylphenyl)propanenitrile include fragrance substances other than the 3-(4-alkylphenyl)propanenitrile represented by Formula (1), surfactants, solvents, antioxidants, and colorants. The additional component is preferably at least one selected from the group consisting of fragrance substances other than the 3-(4-alkylphenyl)propanenitrile represented by Formula (1), surfactants, solvents, antioxidants, and colorants, more preferably at least one selected from the group consisting of fragrance substances other than the 3-(4-alkylphenyl)propanenitrile represented by Formula (1) and solvents, and even more preferably fragrance substances other than the 3-(4-alkylphenyl)propanenitrile represented by Formula (1). That is, a preferred fragrance composition of the present invention contains 3-(4-alkylphenyl)propanenitrile represented by Formula (1) as an active ingredient and contains a fragrance substance other than the 3-(4-alkylphenyl)propanenitrile represented by Formula (1).

The inclusion of a fragrance substance other than the 3-(4-alkylphenyl)propanenitrile represented by Formula (1) allows for adjustment of a scent according to a target product. In addition, the inclusion of a solvent allows for easy dissolution into and impregnation of a target product, making it possible to adjust the intensity of the scent or the durability of the scent.

The fragrance substance other than the 3-(4-alkylphenyl)propanenitrile represented by Formula (1) is not particularly limited as long as it is a known fragrance component, and a wide range of fragrances can be used. For example, one, or two or more of the following fragrance substances can be selected and used at any mixing ratio.

The fragrance substance other than the 3-(4-alkylphenyl)propanenitrile represented by Formula (1) is preferably at least one selected from the group consisting of hydrocarbons, alcohols, esters, phenols, aldehydes, ketones, acetals, ketals, ethers, natural essential oils, natural extracts, and nitriles other than the 3-(4-alkylphenyl)propanenitrile represented by Formula (1).

Among these, the fragrance substance is more preferably at least one selected from the group consisting of hydrocarbons, alcohols, esters, phenols, aldehydes, ketones, acetals, ketals, and ethers, even more preferably at least one selected from the group consisting of alcohols, esters, phenols, and aldehydes, yet even more preferably at least one selected from the group consisting of esters and aldehydes, and yet even more preferably esters. The combination of the 3-(4-alkylphenyl)propanenitrile and these fragrance substances is preferable because a stronger floral scent can be developed.

Examples of the hydrocarbons include limonene, α-pinene, β-pinene, terpinene, cedrene, longifolene, and valencene.

Examples of the alcohols include linalool, citronellol, geraniol, nerol, terpineol, dihydromyrcenol, ethyllinalool, farnesol, nerolidol, cis-3-hexenol, cedrol, menthol, borneol, β-phenylethyl alcohol, benzyl alcohol, phenyl hexanol, 2,2,6-trimethylcyclohexyl-3-hexanol, 1-(2-t-butylcyclohexyloxy)-2-butanol, 4-isopropylcyclohexanemethanol, 4-t-butylcyclohexanol, 4-methyl-2-(2-methylpropyl)tetrahydro-2H-pyran-4-ol, 2-methyl-4-(2,2,3-trimethyl-3-cyclopenten-1-yl)-2-buten-1-ol, 2-ethyl-4-(2,2,3-trimethyl-3-cyclopenten-1-yl)-2-buten-1-ol, isocamphylcyclohexanol, and 3,7-dimethyl-7-methoxyoctan-2-ol.

Examples of the phenols include eugenol, thymol, and vanillin.

Examples of the esters include linalyl formate, citronellyl formate, geranyl formate, n-hexyl acetate, cis-3-hexenyl acetate, linalyl acetate, citronellyl acetate, geranyl acetate, nellyl acetate, terpinyl acetate, nopil acetate, bornyl acetate, isobornyl acetate, o-t-butylcyclohexyl acetate, p-t-butylcyclohexyl acetate, tricyclodecenyl acetate, benzyl acetate, styralyl acetate, cinnamyl acetate, dimethylbenzyl carbinyl acetate, 3-pentyltetrahydropyran-4-yl acetate, citronellyl propionate, tricyclodecenyl propionate, allylcyclohexyl propionate, ethyl-2-cyclohexyl propionate, benzyl propionate, citronellyl butyrate, dimethylbenzylcarbinyl n-butyrate, tricyclodecenyl isobutyrate, methyl-2-nonenoate, methyl benzoate, benzyl benzoate, methyl cinnamate, methyl salicylate, n-hexyl salicylate, cis-3-hexenyl salicylate, geranyl tiglate, cis-3-hexenyl tiglate, methyl jasmonate, methyldihydro jasmonate, methyl-2,4-dihydroxy-3,6-dimethyl benzoate, ethylmethylphenyl glycidate, methyl anthranilate, and fruitate. Among them, at least one selected from the group consisting of linalyl acetate, benzyl acetate, and cinnamyl acetate is preferable, and benzyl acetate is more preferable.

Examples of the aldehydes include n-octanal, n-decanal, n-dodecanal, 2-methylundecanal, 10-undecenal, citronellal, citral, hydroxycitronellal, dimethyl tetrahydrobenzaldehyde, 4-(4-hydroxy-4-methylpentyl)-3-cyclohexene-1-carboaldehyde, 2-cyclohexyl propanal, p-t-butyl-α-methylhydrocinnamic aldehyde, p-t-butylhydrocinnamic aldehyde, p-isopropyl-α-methylhydrocinnamic aldehyde, p-ethyl-α,α-dimethylhydrocinnamic aldehyde, α-amylcinnamic aldehyde, α-hexylcinnamic aldehyde, piperonal, and α-methyl-3,4-methylenedioxyhydrocinnamic aldehyde.

Examples of the ketones include methylheptenone, 4-methylene-3,5,6,6-tetramethyl-2-heptanone, amylcyclopentanone, 3-methyl-2-(cis-2-penten-1-yl)-2-cyclopenten-1-one, methylcyclopentenolone, rose ketone, γ-methylionone, α-ionone, carbone, menthone, camphor, nootkatone, benzylacetone, anysilacetone, methyl-β-naphthylketone, 2,5-dimethyl-4-hydroxy-3(2H)-furanone, maltol, 7-acetyl-1,2,3,4,5,6,7,8-octahydro-1,1,6,7-tetramethyl naphthalene, muscone, civetone, cyclopentadecanone, and cyclohexadecenone.

Examples of the acetals and the ketal include acetoaldehyde ethylphenylpropyl acetal, citraldiethyl acetal, phenylacetoaldehyde glycerin acetal, and ethylacetoacetate ethylene glycol ketal.

Examples of the ethers include anetol, β-naphthylmethyl ether, β-naphthylethyl ether, limonene oxide, rose oxide, 1,8-cineol, racemic or optically active dodecahydro-3a,6,6,9a-tetramethylnaphtho[2,1-b]furane.

Examples of lactones include γ-nonalactone, γ-undecalactone, σ-decalactone, γ-jasmolactone, coumarin, cyclopentadecanolide, cyclohexadecanolide, ambrettolide, ethylene brassylate, and 11-oxahexadecanolide.

Examples of the natural essential oil and the natural extract include those of orange, lemon, bergamot, mandarin, peppermint, spearmint, lavender, chamomile, rosemary, eucalyptus, sage, basil, rose, geranium, jasmine, ylang-ylang, anise, clove, ginger, nutmeg, cardamom, cedar, Japanese cypress, sandalwood, vetiver, patchouli, and labdanum.

Examples of the nitriles other than the 3-(4-alkylphenyl)propanenitrile represented by Formula (1) include citronellylnitrile.

Examples of the solvent include dipropylene glycol, diethyl phthalate, ethylene glycol, propylene glycol, methyl myristate, and triethyl citrate.

Examples of the surfactant include polyoxyethylene lauryl sulfate ether.

The fragrance composition containing the 3-(4-alkylphenyl)propanenitrile represented by General Formula (1) described above can be used as an aromatic component of various products.

The products in which the fragrance composition can be used include: fragrance products, such as perfumes and colognes; cosmetics for hair, such as shampoos, conditioners, hair tonics, hair creams, mousses, gels, pomades, and sprays; cosmetics for skin, such as face lotions, serums, creams, emulsions, facial masks, foundations, face powders, lipsticks, and various types of makeup; various detergents for health and sanitation, such as dish detergents, laundry detergents, softeners, disinfectant detergents, odor eliminating detergents, indoor aromatic agents, furniture care products, glass cleaners, furniture cleaners, floor cleaners, disinfectants, insecticides, bleaches, germicides, and repellents; quasi-drugs, such as toothpaste, mouthwash, bath salts, antiperspirants, and perm solutions; miscellaneous goods such as toilet paper and tissue paper; pharmaceuticals; and food products.

An amount of the fragrance composition blended in the product described above is not particularly limited and can be selected depending on the type, nature, and sensory effect of the product to be perfumed. For example, the amount of the fragrance composition blended in the product is preferably 0.00001 mass% or more, more preferably 0.0001 mass% or more, and even more preferably 0.001 mass% or more. Also, the amount of the fragrance composition blended in the product is preferably 80 mass% or less, more preferably 60 mass% or less, and even more preferably 40 mass% or less.

Note that, when the fragrance composition is used as a fragrance oil, perfume, or the like, the amount of the fragrance composition blended in the product may be 80 mass% or more, or may be 100 mass%.

### [Nitrile composition]

The nitrile composition of the present invention is a nitrile composition containing 3-(4-alkylphenyl)propanenitrile represented by General Formula (1) set forth below and 3-(2-alkylphenyl)propanenitrile represented by General Formula (2) set forth below, wherein the 3-(2-alkylphenyl)propanenitrile represented by General Formula (2) set forth below is contained in an amount of from 0.03 to 0.8 mass% based on the 3-(4-alkylphenyl)propanenitrile represented by Formula (1): 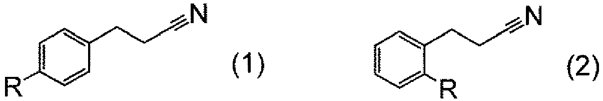 where in Formula (1), R represents an alkyl group having from 2 to 4 carbon atoms, and, in Formula (2), R represents an alkyl group having from 2 to 4 carbon atoms.

The nitrile composition of the present invention contains 3-(4-alkylphenyl)propanenitrile having a floral scent, and a green scent or a fruity scent, as an active ingredient, and contains a small amount of 3-(2-alkylphenyl)propanenitrile represented by Formula (2), and thus is considered to have a more complex and favorable aromatic note, has a strong floral scent, and is excellent in fragrance intensity. Thus, it is useful as an aromatic component of various products. It is also useful as an ingredient for the fragrance composition.

### <3-(4-alkylphenyl)propanenitrile>

In Formula (1), R represents an alkyl group having from 2 to 4 carbon atoms. Specifically, R is preferably at least one selected from the group consisting of an ethyl group, an isopropyl group, an n-butyl group, an isobutyl group, and a tert-butyl group, more preferably at least one selected from the group consisting of an isopropyl group, an n-butyl group, and an isobutyl group, and even more preferably an n-butyl group.

When R is at least one selected from the group consisting of an ethyl group, an isopropyl group, an n-butyl group, an isobutyl group, and a tert-butyl group, the compound has a green scent or a fruity scent, in addition to a floral scent, and can impart a strong floral scent, and therefore is very useful as an active ingredient of the fragrance composition.

When R is at least one selected from the group consisting of an isopropyl group and an isobutyl group, the compound has a green scent and a fruity scent in addition to a floral scent, and can impart a strong floral scent, and therefore is very useful as an active ingredient of the fragrance composition.

When R is an n-butyl group, the compound has a fruity scent and a spicy scent in addition to a floral scent, and can impart a strong floral scent, and therefore is very useful as an active ingredient of the fragrance composition. In addition, when R is an n-butyl group, the intensity and diffusibility of the scent of the fragrance composition can be further significantly improved.

The 3-(4-alkylphenyl)propanenitrile represented by Formula (1) is useful as an active ingredient of the fragrance composition, has a floral scent, and a green scent or a fruity scent, can impart a strong floral scent, and can further impart the intensity and diffusibility of the scent.

When R is an ethyl group, the compound especially has a green scent in addition to a floral scent, and imparts a strong floral scent.

When R is an isopropyl group, the compound especially has a green scent and a fruity scent in addition to a floral scent, and imparts a strong floral scent.

When R is an n-butyl group, the compound especially has a fruity scent and a spicy scent in addition to a floral scent, and imparts a strong floral scent. Furthermore, the intensity and diffusibility of the scent of the fragrance composition can be remarkably improved.

When R is an isobutyl group, the compound especially has a green scent and a fruity scent in addition to a floral scent, and imparts a strong floral scent.

When R is a tert-butyl group, the compound especially has a green scent in addition to a floral scent, and imparts a strong floral scent.

### <3-(2-alkylphenyl)propanenitrile>

In Formula (2), R represents an alkyl group having from 2 to 4 carbon atoms, and when 3-(2-alkylphenyl)propanenitrile is contained in the nitrile composition of the present invention, R in Formula (2) is preferably the same as that of the 3-(4-alkylphenyl)propanenitrile contained in the nitrile composition and is the same as R in Formula (1).

Specifically, R is preferably at least one selected from the group consisting of an ethyl group, an isopropyl group, an n-butyl group, an isobutyl group, and a tert-butyl group, more preferably at least one selected from the group consisting of an isopropyl group, an n-butyl group, and an isobutyl group, and even more preferably an n-butyl group.

When R is at least one selected from the group consisting of an ethyl group, an isopropyl group, an n-butyl group, an isobutyl group, and a tert-butyl group, the compound has a green scent or a fruity scent in addition to a floral scent, and can impart a strong floral scent, and therefore is very useful as an active ingredient of the fragrance composition.

When R is at least one selected from the group consisting of an isopropyl group and an isobutyl group, the compound has a green scent and a fruity scent in addition to a floral scent, and can impart a strong floral scent, and therefore is very useful as an active ingredient of the fragrance composition.

When R is an n-butyl group, the compound has a fruity scent and a spicy scent in addition to a floral scent, and can impart a strong floral scent, and therefore is very useful as an active ingredient of the fragrance composition. In addition, when R is an n-butyl group, the intensity and diffusibility of the scent of the fragrance composition can be further significantly improved.

The nitrile compound of the present invention contains 3-(2-alkylphenyl)propanenitrile represented by Formula (2) in an amount of preferably from 0.03 to 0.8 mass%, more preferably from 0.04 to 0.7 mass%, and even more preferably from 0.2 to 0.7 mass%, based on the 3-(4-alkylphenyl)propanenitrile represented by Formula (1).

When the nitrile composition of the present invention is contained in the fragrance composition, a content of the nitrile composition in the fragrance composition may be appropriately adjusted according to the type of fragrance composition, the type of target aroma, the intensity of the aroma, and the like and is preferably from 0.01 to 90 mass%, more preferably from 0.1 to 50 mass%, even more preferably from 0.5 to 30 mass%, yet even more preferably from 1.0 to 20 mass%, and yet even more preferably from 1.5 to 10 mass%.

### [Use as fragrance]

The 3-(4-alkylphenyl)propanenitrile represented by General Formula (1) set forth below has a floral scent, and a green scent or a fruity scent, and therefore can be used as a fragrance. Use of 3-(4-alkylphenyl)propanenitrile represented by General Formula (1) set forth below as a fragrance is also included in the present invention: 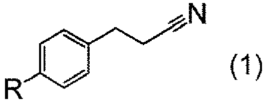 where in Formula (1), R represents an alkyl group having from 2 to 4 carbon atoms.

In Formula (1), R represents an alkyl group having from 2 to 4 carbon atoms. Specifically, R is preferably at least one selected from the group consisting of an ethyl group, an isopropyl group, an n-butyl group, an isobutyl group, and a tert-butyl group, more preferably at least one selected from the group consisting of an isopropyl group, an n-butyl group, and an isobutyl group, and even more preferably an n-butyl group.

When R is at least one selected from the group consisting of an ethyl group, an isopropyl group, an n-butyl group, an isobutyl group, and a tert-butyl group, the compound has a green scent or a fruity scent in addition to a floral scent, and therefore can be more suitably used as a fragrance.

When R is at least one selected from the group consisting of an isopropyl group and an isobutyl group, the compound has a green scent and a fruity scent in addition to a floral scent, and therefore can be even more suitably used as a fragrance.

When R is an n-butyl group, the compound has a fruity scent and a spicy scent in addition to a floral scent, and therefore can be yet even more suitably used as a fragrance.

When R is an ethyl group, the compound especially has a green scent in addition to a floral scent.

When R is an isopropyl group, the compound especially has a green scent and a fruity scent in addition to a floral scent.

When R is an n-butyl group, the compound especially has a fruity scent and a spicy scent in addition to a floral scent.

When R is an isobutyl group, the compound especially has a green scent and a fruity scent in addition to a floral scent.

When R is a tert-butyl group, the compound especially has a green scent in addition to a floral scent.

The 3-(4-alkylphenyl)propanenitrile represented by Formula (1) has a floral scent, and a green scent or a fruity scent. The use of 3-(4-alkylphenyl)propanenitrile represented by Formula (1) therefore imparts a floral scent, and a green scent or a fruity scent, to the product. The use of the compound also makes it possible to improve the intensity and diffusibility of the scent contained in the product.

When R is an ethyl group, the use of the compound especially imparts a green scent in addition to a floral scent.

When R is an isopropyl group, the use of the compound especially imparts a green scent and a fruity scent in addition to a floral scent.

When R is an n-butyl group, the use of the compound especially imparts a fruity scent and a spicy scent in addition to a floral scent. Furthermore, the intensity and diffusibility of the scent contained in the product can be significantly improved.

When R is an isobutyl group, the use of the compound especially imparts a green scent and a fruity scent in addition to a floral scent.

When R is a tert-butyl group, the use of the compound especially imparts a green scent in addition to a floral scent.

The 3-(4-alkylphenyl)propanenitrile represented by Formula (1) can be used as an aromatic component of various products. Note that, in a case where the 3-(4-alkylphenyl)propanenitrile represented by Formula (1) is used as aromatic component for various products, the 3-(4-alkylphenyl)propanenitrile may be used as one component of the fragrance composition.

The products in which the 3-(4-alkylphenyl)propanenitrile represented by Formula (1) can be used include: fragrance products, such as perfumes and colognes; cosmetics for hair, such as shampoos, conditioners, hair tonics, hair creams, mousses, gels, pomades, and sprays; cosmetics for skin, such as face lotions, serums, creams, emulsions, facial masks, foundations, face powders, lipsticks, and various types of makeup; various detergents for health and sanitation, such as dish detergents, laundry detergents, softeners, disinfectant detergents, odor eliminating detergents, indoor aromatic agents, furniture care products, glass cleaners, furniture cleaners, floor cleaners, disinfectants, insecticides, bleaches, germicides, and repellents; quasi-drugs, such as toothpaste, mouthwash, bath salts, antiperspirants, and perm solutions; miscellaneous goods such as toilet paper and tissue paper; pharmaceuticals; and food products.

An amount of the 3-(4-alkylphenyl)propanenitrile of Formula (1) blended in the product described above is not limited. The amount of the 3-(4-alkylphenyl)propanenitrile of Formula (1) blended can be selected depending on the type, nature, and sensory effect of the product to be perfumed. For example, the amount of the 3-(4-alkylphenyl)propanenitrile of Formula (1) blended in the product is preferably 0.00001 mass% or more, more preferably 0.0001 mass% or more, and even more preferably 0.001 mass% or more. Also, the amount of the 3-(4-alkylphenyl)propanenitrile of Formula (1) blended in the product is preferably 80 mass% or less, more preferably 60 mass% or less, and even more preferably 40 mass% or less.

Note that, when the 3-(4-alkylphenyl)propanenitrile represented by Formula (1) is used as a fragrance oil, perfume, or the like, the amount of the 3-(4-alkylphenyl)propanenitrile of Formula (1) blended in the product may be 80 mass% or more, or may be 100 mass%.

### [Scenting method]

Since the 3-(4-alkylphenyl)propanenitrile represented by General Formula (1) has a floral scent, and a green scent or a fruity scent, it can impart a floral scent and, in addition, a green scent or a fruity scent to various products. Also, included in the present invention is a scenting method including imparting a floral scent, and a green scent or a fruity scent, with 3-(4-alkylphenyl)propanenitrile represented by General Formula (1) set forth below.

A method of imparting a floral scent, and, in addition, a green scent or a fruity scent to a product may be coating, spraying, dipping, or the like in a case where the product is a solid and may be mixing in a case where the product is a liquid. In the case of imparting a floral scent, and a green scent or a fruity scent, by the 3-(4-alkylphenyl)propanenitrile represented by Formula (1), the floral scent, and the green scent or the fruity scent, may be imparted to various products by using the 3-(4-alkylphenyl)propanenitrile represented by Formula (1) as one component of the fragrance composition.

In Formula (1), R represents an alkyl group having from 2 to 4 carbon atoms. Specifically, R is preferably at least one selected from the group consisting of an ethyl group, an isopropyl group, an n-butyl group, an isobutyl group, and a tert-butyl group, more preferably at least one selected from the group consisting of an isopropyl group, an n-butyl group, and an isobutyl group, and even more preferably an n-butyl group.

When R is at least one selected from the group consisting of an ethyl group, an isopropyl group, an n-butyl group, an isobutyl group, and a tert-butyl group, the compound can impart a green scent or a fruity scent in addition to a floral scent.

When R is at least one selected from the group consisting of an isopropyl group and an isobutyl group, the compound can impart a green scent and a fruity scent in addition to a floral scent.

When R is an n-butyl group, the compound can impart a fruity scent and a spicy scent in addition to a floral scent.

Since the 3-(4-alkylphenyl)propanenitrile represented by Formula (1) has a floral scent, and a green scent or a fruity scent, it can impart a floral scent, and a green scent or a fruity scent to various products.

When R is an ethyl group, the compound especially imparts a green scent to the product in addition to a floral scent.

When R is an isopropyl group, the compound especially imparts a green scent and a fruity scent to the product in addition to a floral scent.

When R is an n-butyl group, the compound especially imparts a fruity scent and a spicy scent to the product in addition to a floral scent. Furthermore, the intensity and diffusibility of the scent contained in the product can be significantly improved.

When R is an isobutyl group, the compound especially imparts a green scent and a fruity scent to the product in addition to a floral scent.

When R is a tert-butyl group, the compound especially imparts a green scent to the product in addition to a floral scent.

The products to which a floral scent, and a green scent or a fruity scent, can be imparted by the 3-(4-alkylphenyl)propanenitrile represented by Formula (1) include: fragrance products, such as perfumes and colognes; cosmetics for hair, such as shampoos, conditioners, hair tonics, hair creams, mousses, gels, pomades, and sprays; cosmetics for skin, such as face lotions, serums, creams, emulsions, facial masks, foundations, face powders, lipsticks, and various types of makeup; various detergents for health and sanitation, such as dish detergents, laundry detergents, softeners, disinfectant detergents, odor eliminating detergents, indoor aromatic agents, furniture care products, glass cleaners, furniture cleaners, floor cleaners, disinfectants, insecticides, bleaches, germicides, and repellents; quasi-drugs, such as toothpaste, mouthwash, bath salts, antiperspirants, and perm solutions; miscellaneous goods such as toilet paper and tissue paper; pharmaceuticals; and food products.

An amount of the 3-(4-alkylphenyl)propanenitrile of Formula (1) blended in the product described above is not limited. The amount of the 3-(4-alkylphenyl)propanenitrile of Formula (1) blended can be selected depending on the type, nature, and sensory effect of the product to be perfumed. For example, the amount of the 3-(4-alkylphenyl)propanenitrile of Formula (1) blended in the product is preferably 0.00001 mass% or more, more preferably 0.0001 mass% or more, and even more preferably 0.001 mass% or more. Also, the amount of the 3-(4-alkylphenyl)propanenitrile of Formula (1) blended in the product is preferably 80 mass% or less, more preferably 60 mass% or less, and even more preferably 40 mass% or less.

Note that, when the 3-(4-alkylphenyl)propanenitrile represented by Formula (1) is used as a fragrance oil, perfume, or the like, the amount of the 3-(4-alkylphenyl)propanenitrile of Formula (1) blended in the product may be 80 mass% or more, or may be 100 mass%.

### [Method for producing 3-(4-alkylphenyl)propanenitrile]

A method for producing the 3-(4-alkylphenyl)propanenitrile contained as the active ingredient in the fragrance composition of the present invention is not limited, but it is preferable to obtain the 3-(4-alkylphenyl)propanenitrile by a production method described set forth below. Note that the 3-(4-alkylphenyl)propanenitrile obtained in the production method described below is also preferably used as a fragrance as described above and is also preferably used in the scenting method. Furthermore, the production method described below is also included in the present invention.

The method for producing 3-(4-alkylphenyl)propanenitrile of the present invention is a method for producing 3-(4-alkylphenyl)propanenitrile including, in this order: a step of converting, via a condensation reaction, 4-alkylbenzaldehyde represented by Formula (3) as a raw material and acetonitrile into cinnamonitrile represented by Formula (4) set forth below; and a hydrogenation step: 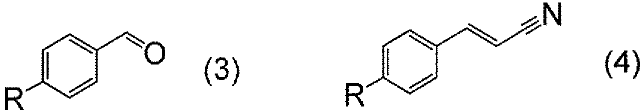 where in Formulae (3) and (4), R represents an alkyl group having from 2 to 4 carbon atoms.

A reaction in the case of producing 3-(4-alkylphenyl)propanenitrile by the production method is represented by the following formula: 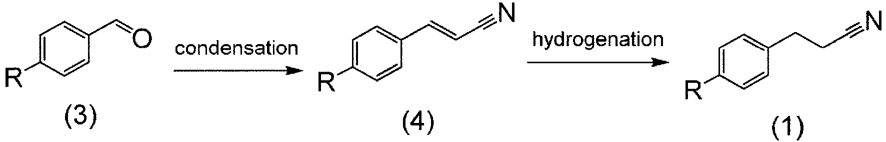 wherein R represents an alkyl group having from 1 to 4 carbon atoms.

### <Condensation step>

The production method includes a condensation step of subjecting 4-alkylbenzaldehyde and acetonitrile to dehydration condensation: 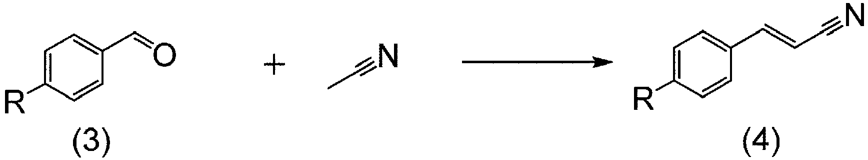 wherein R represents an alkyl group having from 1 to 4 carbon atoms.

In the condensation reaction in this step, a basic compound is preferably used as a catalyst.

Examples of the basic compound to be used as a catalyst include sodium hydroxide, potassium hydroxide, sodium bicarbonate, and mixtures of these. An amount of the basic compound is preferably 0.05 equivalents or more, more preferably 0.1 equivalents or more, and even more preferably 0.2 equivalents or more, and preferably 3 equivalents or less, more preferably 1 equivalent or less, and even more preferably 0.7 equivalents or less per equivalent of the raw material dimethylbenzaldehyde.

An amount of the acetonitrile to be added is preferably 1 equivalent or more, and more preferably 2 equivalents or more, and preferably 10 equivalents or less, and more preferably 7 equivalents or less, per equivalent of 4-alkylbenzaldehyde, which is a raw material. 4-Alkylbenzaldehyde is preferably added sequentially or continuously over time, and for example, is preferably added dropwise.

The condensation reaction in this step can be carried out using acetonitrile as a solvent, but may be carried out in the presence of another solvent. The solvent to be used is not particularly limited as long as it does not inhibit the condensation reaction, and examples thereof include: alcohols, such as methanol, ethanol, and isopropanol; and hydrocarbon solvents including: aliphatic hydrocarbons, such as pentane, hexane, isopentane, heptane, octane, and isooctane; and aromatic hydrocarbons, such as benzene, toluene, ethylbenzene, and xylene. One of these may be used individually, or two or more of these may be used in combination.

The reaction temperature in the condensation reaction in this step is not particularly limited but is preferably 0°C or higher and more preferably 10°C or higher from the viewpoint of the reaction rate, and preferably 100°C or lower, more preferably 70°C or lower, and even more preferably 50°C or lower from the viewpoint of suppressing side reactions.

The reaction time is not particularly limited as long as the condensation is sufficiently performed, and is preferably 10 minutes or more, more preferably 30 minutes or more, and even more preferably 1 hour or more, and is preferably 24 hours or less, more preferably 12 hours or less, even more preferably 6 hours or less, and yet even more preferably 3 hours or less.

The reaction is to be stopped by neutralization; for example, the reaction can be stopped by adding an acid, such as acetic acid.

A method for isolating the intermediate represented by Formula (3) described above from the solution after completion of the reaction is not particularly limited, and it may be performed by appropriately combining liquid separation, extraction operation, and purification by distillation. Alternatively, the intermediate may be used in the subsequent hydrogenation step without isolation.

As a method in the case of isolating the intermediate, for example, a low-polar or nonpolar organic solvent is added to the solution after completion of the reaction to transfer the intermediate to an oil phase; the resulting oil phase is dried, for example, with magnesium sulfate; a filtrate obtained by filtration is then concentrated, and further purified by distillation; and the intermediate can be isolated accordingly.

### <Hydrogenation step>

The production method described above includes a hydrogenation step after the condensation step and produces the 3-(4-alkylphenyl)propanenitrile represented by Formula (1) described above.

This is a step in which the intermediate of the above-described Formula (4) obtained by the condensation step is hydrogenated to yield the target product 3-(4-alkylphenyl)propanenitrile represented by Formula (1) described above. The hydrogenation method is not particularly limited but can be carried out by a known method using a hydrogenation catalyst.

The hydrogenation catalyst is not particularly limited, and a known catalyst can be used. Examples of the known catalyst that can be used include: a supported heterogeneous hydrogenation catalyst in which a metal, such as Ni, Pt, Pd, or Ru, is supported on carbon, silica, alumina, diatomaceous earth, or the like; what is called a Ziegler-type hydrogenation catalyst prepared using an organic acid salt of Ni, Co, Fe, Cr, or the like, or a transition metal salt, such as an acetylacetone salt, and a reducing agent, such as organoaluminum; and a homogeneous hydrogenation catalyst of what is called an organometallic complex or the like, such as an organometallic compound of Ti, Ru, Rh, Zr, or the like.

The temperature of the hydrogenation reaction in this step is preferably 0°C or higher, more preferably 10°C or higher, and even more preferably 20°C or higher, and is preferably 150°C or lower and more preferably 100°C or lower, from the viewpoints of the reactivity and suppressing side reactions.

The pressure of hydrogen used in the hydrogenation reaction is preferably 0.01 MPaG or more, more preferably 0.03 MPaG or more, and even more preferably 0.05 MPaG or more, and is preferably 10 MPaG or less, more preferably 3 MPaG or less, even more preferably 1 MPaG or less, and yet even more preferably 0.5 MPaG or less.

The reaction time is not particularly limited, and is preferably 3 minutes or more, more preferably 10 minutes or more, and even more preferably 30 minutes or more, and is preferably 24 hours or less, more preferably 12 hours or less, and even more preferably 8 hours or less.

The hydrogenation reaction may be performed in the presence of a solvent. The solvent to be used is not particularly limited as long as it does not inhibit the hydrogenation reaction, and examples thereof includes: alcohols, such as methanol, ethanol, and isopropanol; and hydrocarbon solvents including: aliphatic hydrocarbons, such as pentane, hexane, isopentane, heptane, octane, and isooctane; and aromatic hydrocarbons, such as benzene, toluene, ethylbenzene, and xylene. One of these may be used individually, or two or more of these may be used in combination.

A method for purifying the target product 3-(4-alkylphenyl)propanenitrile represented by Formula (1), from the solution after completion of the reaction is not particularly limited, and a known method may be appropriately selected and performed. Specifically, examples of the method include filtration, chromatography, and purification by distillation, and purification by appropriately combining these can provide target 3-(4-alkylphenyl)propanenitrile of high purity.

### Examples

The present invention will be described in detail based on Examples presented below, but the present invention is not limited to these Examples.

### <Analysis of composition>

The composition in each step described later, the composition of the product, and the composition of the composition were determined using gas chromatography (GC-2010 Plus, available from Shimadzu Corporation).

A capillary column HR-1701 (0.32 mm φ in inner diameter and 30 m in length) available from Shinwa Chemical Industries LTD. was used. In the temperature raising program, the temperature was raised from 100°C to 280°C at a rate of 5°C/min and maintained for 30 minutes.

### <NMR spectral analysis>

Instrument: Bruker AVANCE NEO 400 MHz
Solvent: deuterated chloroform (CDCl₃)
Measurement mode: ¹H, ¹³C
Internal standard substance: tetramethylsilane (TMS)

### <Evaluation of scent/aromatic note>

The scent and aromatic note of nitrile compounds and fragrance compositions obtained in examples were evaluated by a method in which filter paper 8 mm in width and 15 cm in length was impregnated with the compound and the composition, and an expert panelist smelled the filter paper.

### [Production of 3-(4-alkylphenyl)propanenitrile]

### Example 1 (Production of 3-(4-ethylphenyl)propanenitrile)

### (Condensation step)

Acetonitrile (available from FUJIFILM Wako Pure Chemical Corporation, 150.5 g) and potassium hydroxide (available from FUJIFILM Wako Pure Chemical Corporation, 24.4 g) were charged into a round-bottom flask having an internal volume of 500 mL and equipped with a stirrer, a thermometer, and a dropping funnel, the temperature was raised to 50°C with stirring, and then 4-ethylbenzaldehyde (available from MITSUBISHI GAS CHEMICAL COMPANY, INC., 100.0 g) was added dropwise thereto over 2 hours. After completion of the dropwise addition, the mixture was maintained at 50°C for 2 hours, and the reaction was completed. Acetic acid was added to neutralize the mixture, then water and heptane were added, and the aqueous phase was separated by liquid separation. Heptane was then distilled off, and a crude intermediate solution was obtained. The crude intermediate solution was subjected to simple distillation to obtain 3-(4-ethylphenyl)propenenitrile as a mixture of isomers (42.0 g, total purity: 99.3%).

### (Hydrogenation step)

The 3-(4-ethylphenyl)propenenitrile (41.0 g) obtained in the condensation step, 2-propanol (41.0 g), and a 5% palladium-carbon catalyst (available from N. E. CHEMCAT CORPORATION, water-containing product, PE type, 0.8 g) were charged into a 200-mL stainless steel autoclave equipped with a magnetic induction stirrer and capable of controlling the internal temperature by a jacket, and stirred at 40°C and a hydrogen pressure of 0.4 MPa for 5 hours to perform a hydrogenation reaction. The reaction liquid was filtered to remove the catalyst, heptane was added, and the aqueous phase was separated by liquid separation. Heptane was then distilled off, and a crude product was obtained. The crude product was purified by column chromatography (silica gel, hexane:ethyl acetate = 95:5) to obtain 3-(4-ethylphenyl)propanenitrile (20.0 g, purity: 99.0%) as a colorless transparent liquid. Note that 3-(2-ethylphenyl)propanenitrile was contained in an amount of 0.04 mass% based on the 3-(4-ethylphenyl)propanenitrile.

### (Scent/aromatic note)

Floral, Green, Mashroomy, Earthy

### (NMR spectrum)

¹H-NMR(400 MHz, CDCl₃)δ1.23(3H, t, J=7.6 Hz), 2.57-2.66(4H, m), 2.92(2H, t, J=7.4 Hz), 7.13-7.18(4H, m)
¹³C-NMR(100 MHz, CDCl₃)δ15.5, 19.4, 28.5, 31.2, 119.2, 128.2, 128.4, 135.3, 143.3

### Example 2 (Production of 3-(4-isopropylphenyl)propanenitrile)

### (Condensation step)

Acetonitrile (available from FUJIFILM Wako Pure Chemical Corporation, 135.1 g) and potassium hydroxide (available from FUJIFILM Wako Pure Chemical Corporation, 22.2 g) were charged into a round-bottom flask having an internal volume of 500 mL and equipped with a stirrer, a thermometer, and a dropping funnel, the temperature was raised to 50°C with stirring, and then 4-isopropylbenzaldehyde (available from MITSUBISHI GAS CHEMICAL COMPANY, INC., 100.0 g) was added dropwise thereto over 2 hours. After completion of the dropwise addition, the mixture was maintained at 50°C for 2 hours, and the reaction was completed. Acetic acid was added to neutralize the mixture, then water and heptane were added, and the aqueous phase was separated by liquid separation. Heptane was then distilled off, and a crude intermediate solution was obtained. The crude intermediate solution was subjected to simple distillation to obtain 3-(4-isopropylphenyl)propenenitrile as a mixture of isomers (53.0 g, total purity: 98.3%).

### (Hydrogenation step)

The 3-(4-isopropylphenyl)propenenitrile (51.0 g) obtained in the condensation step, 2-propanol (51.0 g), and a 5% palladium-carbon catalyst (available from N. E. CHEMCAT CORPORATION, water-containing product, PE type, 0.5 g) were charged into a 200-mL stainless steel autoclave equipped with a magnetic induction stirrer and capable of controlling the internal temperature by a jacket, and stirred at 40°C and a hydrogen pressure of 0.4 MPa for 5 hours to perform a hydrogenation reaction. The reaction liquid was filtered to remove the catalyst, heptane was added, and the aqueous phase was separated by liquid separation. Heptane was then distilled off, and a crude product was obtained. The crude product was purified by column chromatography (silica gel, hexane:ethyl acetate = 95:5) to obtain 3-(4-isopropylphenyl)propanenitrile (25.0 g, purity: 99.0%) as a colorless transparent liquid. Note that 3-(2-isopropylphenyl)propanenitrile was contained in an amount of 0.2 mass% based on the 3-(4-isopropylphenyl)propanenitrile.

### (Scent/aromatic note)

Floral, Green, Fruity, Rose, Muguet

### (NMR spectrum)

¹H-NMR(400 MHz, CDCl₃)δ1.24(6H, d, J=6.9 Hz), 2.6(2H, t, J=7.4), 2.86-2.94(3H, m), 7.14-7.21(4H, m)
¹³C-NMR(100 MHz, CDCl₃)δ19.4, 24.0, 31.2, 33.8, 119.3, 126.9, 128.2, 135.4, 147.9

### Example 3 (Production of 3-(4-n-butylphenyl)propanenitrile)

### (Condensation step)

Acetonitrile (available from FUJIFILM Wako Pure Chemical Corporation, 123.4 g) and potassium hydroxide (available from FUJIFILM Wako Pure Chemical Corporation, 28.5 g) were charged into a round-bottom flask having an internal volume of 500 mL and equipped with a stirrer, a thermometer, and a dropping funnel, the temperature was raised to 50°C with stirring, and then 4-n-butylbenzaldehyde (available from MITSUBISHI GAS CHEMICAL COMPANY, INC., 100.0 g) was added dropwise thereto over 2 hours. After completion of the dropwise addition, the mixture was maintained at 50°C for 2 hours, and the reaction was completed. Acetic acid was added to neutralize the mixture, then water and heptane were added, and the aqueous phase was separated by liquid separation. Heptane was then distilled off, and a crude intermediate solution was obtained. The crude intermediate solution was subjected to simple distillation (from 160 to 162°C/5 torr) to obtain 3-(4-n-butylphenyl)propenenitrile as a mixture of isomers (64.0 g, total purity: 98.5%).

### (Hydrogenation step)

The 3-(4-n-butylphenyl)propenenitrile (64.0 g) obtained in the condensation step, 2-propanol (64.0 g), and a 5% palladium-carbon catalyst (available from N. E. CHEMCAT CORPORATION, water-containing product, PE type, 1.2 g) were charged into a 200-mL stainless steel autoclave equipped with a magnetic induction stirrer and capable of controlling the internal temperature by a jacket, and stirred at 40°C and a hydrogen pressure of 0.4 MPa for 5 hours to perform a hydrogenation reaction. The reaction liquid was filtered to remove the catalyst, heptane was added, and the aqueous phase was separated by liquid separation. Heptane was then distilled off, and a crude product was obtained. The crude product was rectified at 10 torr using a rectification column having a theoretical plate number of 20 stages to obtain 3-(4-n-butylphenyl)propanenitrile (46.0 g, purity: 99.0%) as a colorless transparent liquid, as a fraction at from 160 to 166°C. Note that 3-(2-n-butylphenyl)propanenitrile was contained in an amount of 0.7 mass% based on the 3-(4-n-butylphenyl)propanenitrile.

### (Scent/aromatic note)

Spicy, Floral, Fuity, Lactonic

### (NMR spectrum)

¹H-NMR(400 MHz, CDCl₃)δ0.92(3H, t, J=7.3 Hz), 1.30-1.40(2H, m), 1.55-1.62(2H, m), 2.57-2.61(4H, m), 2.92(2H, t, J=7.4 Hz), 7.11-7.16(4H, m)
¹³C-NMR(100 MHz, CDCl₃)δ 13.9, 19.4, 22.3, 31.2, 33.6, 35.2, 119.2, 128.1, 128.9, 135.2, 141.9

### Example 4 (Production of 3-(4-isobutylphenyl)propanenitrile)

### (Condensation step)

Acetonitrile (available from FUJIFILM Wako Pure Chemical Corporation, 123.5 g) and potassium hydroxide (available from FUJIFILM Wako Pure Chemical Corporation, 20.5 g) were charged into a round-bottom flask having an internal volume of 500 mL and equipped with a stirrer, a thermometer, and a dropping funnel, the temperature was raised to 50°C with stirring, and then 4-isobutylbenzaldehyde (available from MITSUBISHI GAS CHEMICAL COMPANY, INC., 100.0 g) was added dropwise thereto over 2 hours. After completion of the dropwise addition, the mixture was maintained at 50°C for 2 hours, and the reaction was completed. Acetic acid was added to neutralize the mixture, then water and heptane were added, and the aqueous phase was separated by liquid separation. Heptane was then distilled off, and a crude intermediate solution was obtained. The crude intermediate solution was subjected to simple distillation to obtain 3-(4-isobutylphenyl)propenenitrile as a mixture of isomers (55.0 g, total purity: 97.6%).

### (Hydrogenation step)

The 3-(4-isobutylphenyl)propenenitrile (53.0 g) obtained in the condensation step, 2-propanol (53.0 g), and a 5% palladium-carbon catalyst (available from N. E. CHEMCAT CORPORATION, water-containing product, PE type, 1.0 g) were charged into a 200-mL stainless steel autoclave equipped with a magnetic induction stirrer and capable of controlling the internal temperature by a jacket, and stirred at 40°C and a hydrogen pressure of 0.4 MPa for 5 hours to perform a hydrogenation reaction. The reaction liquid was filtered to remove the catalyst, heptane was added, and the aqueous phase was separated by liquid separation. Heptane was then distilled off, and a crude product was obtained. The crude product was purified by column chromatography (silica gel, hexane:ethyl acetate = 95:5) to obtain 3-(4-isobutylphenyl)propanenitrile (22.0 g, purity: 99.2%) as a colorless transparent liquid. Note that 3-(2-isobutylphenyl)propanenitrile was contained in an amount of 0.2 mass% based on the 3-(4-isobutylphenyl)propanenitrile.

### (Scent/aromatic note)

Floral, Green, Fruity, Green, Muguet

### (NMR spectrum)

¹H-NMR(400 MHz, CDCl₃)δ0.90(6H, d, J=6.6 Hz), 1.79-1.90(1H, m), 2.45(2H, d, J=7.2 Hz), 2.60(2H, t, J=7.5 Hz), 2.93(2H, t, J=7.5 Hz), 7.09-7.14(4H, m)
¹³C-NMR(100 MHz, CDCl₃)δ19.4, 22.3, 30.2, 31.3, 45.0, 119.2, 128.0, 129.6, 135.3, 140.7

### Example 5 (Production of 3-(4-tert-butylphenyl)propanenitrile)

### (Condensation step)

Acetonitrile (available from FUJIFILM Wako Pure Chemical Corporation, 123.3 g) and potassium hydroxide (available from FUJIFILM Wako Pure Chemical Corporation, 24.4 g) were charged into a round-bottom flask having an internal volume of 500 mL and equipped with a stirrer, a thermometer, and a dropping funnel, the temperature was raised to 50°C with stirring, and then 4-tert-butylbenzaldehyde (available from Tokyo Chemical Industry Co., Ltd., 50.0 g) was added dropwise thereto over 1 hour. After completion of the dropwise addition, the mixture was maintained at 50°C for 2 hours, and the reaction was completed. Acetic acid was added to neutralize the mixture, then water and heptane were added, and the aqueous phase was separated by liquid separation. Heptane was then distilled off, and a crude intermediate solution was obtained. The crude intermediate solution was subjected to simple distillation to obtain 3-(4-tert-butylphenyl)propenenitrile as a mixture of isomers (19.7 g, total purity: 94.1%).

### (Hydrogenation step)

The 3-(4-tert-butylphenyl)propenenitrile (19.7 g) obtained in the condensation step, methanol (40.0 g), and a 5% palladium-carbon catalyst (available from N. E. CHEMCAT CORPORATION, water-containing product, PE type, 0.4 g) were charged into a 200-mL stainless steel autoclave equipped with a magnetic induction stirrer and capable of controlling the internal temperature by a jacket, and stirred at 40°C and a hydrogen pressure of 0.4 MPa for 5 hours to perform a hydrogenation reaction. The reaction liquid was filtered to remove the catalyst, heptane was added, and the aqueous phase was separated by liquid separation. Heptane was then distilled off, and a crude product was obtained. The crude product was purified by column chromatography (silica gel, hexane:ethyl acetate = 95:5) to obtain 3-(4-tert-butylphenyl)propanenitrile (6.4 g, purity: 98.0%) as a colorless transparent liquid. Note that 3-(2-tert-butylphenyl)propanenitrile was contained in an amount of 0.6 mass% based on the 3-(4-tert-butylphenyl)propanenitrile.

### (Scent/aromatic note)

Floral, Green, Woody, Peony, Musky

### (NMR spectrum)

¹H-NMR(400 MHz, CDCl₃)δ1.31(9H, s), 2.60(2H, t, J=7.5 Hz), 2.93(2H, t, J=7.5 Hz), 7.16(2H, d, J=8.4 Hz), 7.35(2H, d, J=8.4)
¹³C-NMR(100 MHz, CDCl₃)δ19.3, 31.1, 31.3, 34.5, 119.3, 125.8, 127.9, 135.0, 150.2

### Comparative Example 1 (Production of 3-(4-methylphenyl)propanenitrile)

### (Condensation step)

Acetonitrile (available from FUJIFILM Wako Pure Chemical Corporation, 167.8 g) and potassium hydroxide (available from FUJIFILM Wako Pure Chemical Corporation, 38.47 g) were charged into a round-bottom flask having an internal volume of 500 mL and equipped with a stirrer, a thermometer, and a dropping funnel, the temperature was raised to 50°C with stirring, and then 4-methylbenzaldehyde (available from MITSUBISHI GAS CHEMICAL COMPANY, INC., 100.0 g) was added dropwise thereto over 2 hours. After completion of the dropwise addition, the mixture was maintained at 50°C for 2 hours, and the reaction was completed. Acetic acid was added to neutralize the mixture, then water and heptane were added, and the aqueous phase was separated by liquid separation. Heptane was then distilled off, and a crude intermediate solution (120.0 g, purity: 81.6%) was obtained as a solid. The crude intermediate was used as it was in the hydrogenation step.

### (Hydrogenation step)

The crude intermediate containing 3-(4-methylphenyl)propenenitrile (50.0 g) obtained in the condensation step, methanol (50.0 g), and a 5% palladium-carbon catalyst (available from N. E. CHEMCAT CORPORATION, water-containing product, PE type, 1.0 g) were charged into a 200-mL stainless steel autoclave equipped with a magnetic induction stirrer and capable of controlling the internal temperature by a jacket, and stirred at 40°C and a hydrogen pressure of 0.4 MPa for 5 hours to perform a hydrogenation reaction. The reaction liquid was filtered to remove the catalyst, heptane was added, and the aqueous phase was separated by liquid separation. Heptane was then distilled off, and a crude product was obtained. The crude product was purified by column chromatography (silica gel, hexane:ethyl acetate = 95:5) to obtain 3-(4-methylphenyl)propanenitrile (18.0 g, purity: 98.8%) as a colorless transparent liquid. Note that 3-(2-methylphenyl)propanenitrile was contained in an amount of 1.0 mass% based on the 3-(4-methylphenyl)propanenitrile.

### (Scent/aromatic note)

Green, Herbal, Floral, Spicy, Hyacinth

### (NMR spectrum)

¹H-NMR(400 MHz, CDCl₃)δ2.33(3H, s), 2.59(2H, t, J=7.4), 2.91(2H, t, J=7.4 Hz), 7.10-7.15(4H, m)
¹³C-NMR(100 MHz, CDCl₃)δ19.5, 21.0, 31.2, 119.2, 128.1, 129.5, 135.0, 136.9

It is found that the 3-(4-alkylphenyl)propanenitriles obtained in examples can be suitably used as a fragrance as having a green scent or a fruity scent in addition to a floral scent. Further, it is found that the 3-(4-alkylphenyl)propanenitriles obtained in examples can be suitably used in a scenting method including imparting a green scent or a fruity scent in addition to a floral scent.

In particular, it is found that the 3-(4-isopropylphenyl)propanenitrile and 3-(4-isobutylphenyl)propanenitrile obtained in Examples 2 and 4 have a green scent and a fruity scent in addition to a floral scent, and thus can be more suitably used as a fragrance and can be more suitably used in a scenting method.

In addition, it is found that the 3-(4-n-butylphenyl)propanenitrile obtained in Example 3 has a fruity scent and a spicy scent in addition to a floral scent, and thus can be even more suitably used as a fragrance and can be even more suitably used in a scenting method.

On the other hand, the 3-(4-methylphenyl)propanenitrile obtained in Comparative Example 1 had a green scent and a herbal scent, and had a weak floral scent.

### [Fragrance composition]

### Example 6 (Apple cinnamon-like fragrance composition)

To the fragrance composition base shown in Table 1, the 3-(4-n-butylphenyl)propanenitrile obtained in Example 3 was added so as to attain 1.50 mass%, and the scent/aromatic note was evaluated.

### Comparative Example 2 (Apple cinnamon-like fragrance composition)

To the fragrance composition base shown in Table 1, CINNAMALVA ((E)-cinnamyl nitrile) was added so as to attain 1.50 mass%, and the scent/aromatic note was evaluated.

**[Table 1]**

| Table 1 | | (mass%) |
|---|---|---|
| | Example 6 | Comparative Example 2 |
| 3-(4-n-Butylphenyl)propanenitrile (Example 3) | 1.50 | - |
| CINNAMALVA | - | 1.50 |
| 2,3,5-TRIMETHYL PYRAZINE | 0.10 | 0.10 |
| ALDEHYDE C-14 | 3.50 | 3.50 |
| ALLYL CAPROATE | 1.00 | 1.00 |
| ALLYL CYCLOHEXYL PROPIONATE | 3.00 | 3.00 |
| ALLYL IONONE | 0.10 | 0.10 |
| ALLYL OENANTHATE | 6.00 | 6.00 |
| CINNAMIC ACETATE | 27.00 | 27.00 |
| CINNAMYL PROPIONATE | 6.50 | 6.50 |
| CIS-3-HEXENOL | 0.10 | 0.10 |
| CIS-3-HEXENYL ACETATE | 0.10 | 0.10 |
| CYCLAMEN ALDEHYDE | 2.00 | 2.00 |
| CYCLAPROP | 1.00 | 1.00 |
| D.M.B.C. BUTYRATE | 1.00 | 1.00 |
| DIHYDRO MYRCENOL | 2.50 | 2.50 |
| DPG | 20.00 | 20.00 |
| ETHYL CAPRATE | 0.60 | 0.60 |
| ETHYL CAPROATE | 1.00 | 1.00 |
| ETHYL-2-METHYL BUTYRATE | 1.00 | 1.00 |
| ETHYLENE BRASSYLATE | 5.00 | 5.00 |
| FRUCTONE | 10.00 | 10.00 |
| FURANEOL | 0.04 | 0.04 |
| HEXYL ACETATE | 1.00 | 1.00 |
| IONONE ALPHA | 0.50 | 0.50 |
| LIGUSTRAL | 0.50 | 0.50 |
| MALTOL | 0.16 | 0.16 |
| ORANGE TERPENES | 1.50 | 1.50 |
| PRENYL ACETATE | 0.30 | 0.30 |
| VANILLIN | 1.00 | 1.00 |
| VERDOX | 2.00 | 2.00 |
| Total | 100.00 | 100.00 |

The fragrance composition of Example 6 had a strong floral and fruity scent and was excellent in intensity and diffusibility of the scent, as compared with the fragrance composition of Comparative Example 2.

### Example 7 (Herb-like fragrance composition)

To the fragrance composition base shown in Table 2, the 3-(4-isopropylphenyl)propanenitrile obtained in Example 2 was added so as to attain 10.0 mass%, and the scent/aromatic note was evaluated.

### Example 8 (Herb-like fragrance composition)

To the fragrance composition base shown in Table 2, the 3-(4-isobutylphenyl)propanenitrile obtained in Example 4 was added so as to attain 10.0 mass%, and the scent/aromatic note was evaluated.

### Comparative Example 3 (Herb-like fragrance composition)

To the fragrance composition base shown in Table 2, 10.0 mass% of dipropylene glycol (DPG) as a solvent was added so as to attain 18.0 mass%, and the scent/aromatic note was evaluated.

**[Table 2]**

| Table 2 | | | (mass%) |
|---|---|---|---|
| | Example 7 | Example 8 | Comparative Example 3 |
| 3-(4-Isopropylphenyl)propanenitrile (Example 2) | 10.0 | - | - |
| 3-(4-Isobutylphenyl)propanenitrile (Example 4) | - | 10.0 | - |
| DPG | 8.0 | 8.0 | 18.0 |
| ALDEHYDE C-14 | 3.0 | 3.0 | 3.0 |
| AMBERONE | 8.0 | 8.0 | 8.0 |
| ANETHOL | 3.0 | 3.0 | 3.0 |
| BORNEOL LAEVO CRIST | 2.6 | 2.6 | 2.6 |
| CAMPHOR | 5.4 | 5.4 | 5.4 |
| CITRONELLYL ACETATE | 1.0 | 1.0 | 1.0 |
| DIHYDRO MYRCENOL | 6.0 | 6.0 | 6.0 |
| DIMETHYL OCTANOL | 1.2 | 1.2 | 1.2 |
| EUCALYPTOL | 15.0 | 15.0 | 15.0 |
| HEXYL SALICYLATE | 10.0 | 10.0 | 10.0 |
| ISO BORNYL ACETATE | 1.8 | 1.8 | 1.8 |
| ISOANANAT | 4.0 | 4.0 | 4.0 |
| LEMONILE | 2.0 | 2.0 | 2.0 |
| LIGUSTRAL | 0.6 | 0.6 | 0.6 |
| PARA CYMENE | 3.0 | 3.0 | 3.0 |
| PINENE PETA | 6.0 | 6.0 | 6.0 |
| TAZINAL 10%TEC 10%DPG | 0.4 | 0.4 | 0.4 |
| TERPINEOL | 1.6 | 1.6 | 1.6 |
| TETRA HYDRO LINALOOL | 4.0 | 4.0 | 4.0 |
| THYMOL CRYSTALS | 0.2 | 0.2 | 0.2 |
| UNDECAVERTOL | 1.0 | 1.0 | 1.0 |
| VERDOX | 2.0 | 2.0 | 2.0 |
| VIRDINE | 0.2 | 0.2 | 0.2 |
| Total | 100.0 | 100.0 | 100.0 |

The fragrance composition of Example 7 had a strong middle note floral scent and a strong lilac-like floral feeling, as compared with the fragrance composition of Comparative Example 3. The fragrance composition of Example 8 had a strong middle note floral scent and a strong geranium-like floral feeling, as compared with the fragrance composition of Comparative Example 3.

### Example 9 (Fragrance composition suited for softener)

To the fragrance composition base shown in Table 3, the 3-(4-isopropylphenyl)propanenitrile obtained in Example 2 was added so as to attain 6.5 mass%, and the scent/aromatic note was evaluated.

### Comparative Example 4 (Fragrance composition suited for softener)

To the fragrance composition base shown in Table 3, 6.5 mass% of dipropylene glycol (DPG) as a solvent was added so as to attain 14.3 mass%, and the scent/aromatic note was evaluated.

**[Table 3]**

| Table 3 | | (mass%) |
|---|---|---|
| | Example 9 | Comparative Example 4 |
| 3-(4-Isopropylphenyl)propanenitrile (Example 2) | 6.5 | - |
| DPG | 7.8 | 14.3 |
| ALDEHYDE C-11 UNDECYLENIC | 0.6 | 0.6 |
| ALDEHYDE C-12 LAURIC | 2.4 | 2.4 |
| ALDEHYDE C-12 MNA | 0.8 | 0.8 |
| ALDEHYDE C-14 | 1.7 | 1.7 |
| ANETHOL | 0.2 | 0.2 |
| BENZYL ACETATE | 18.0 | 18.0 |
| COUMARIN | 5.4 | 5.4 |
| DAMASCONE DELTA | 0.2 | 0.2 |
| ETHYL VANILLIN | 0.6 | 0.6 |
| EUGENOL | 2.7 | 2.7 |
| GERANIOL | 8.5 | 8.5 |
| GERANYL ACETATE | 0.3 | 0.3 |
| HEXYL SALICYLATE | 0.3 | 0.3 |
| LIGUSTRAL | 1.2 | 1.2 |
| LINALOOL | 18.0 | 18.0 |
| METHYL ANTHRANILATE | 0.2 | 0.2 |
| METHYL BENZOATE | 0.5 | 0.5 |
| METHYL IONONE GAMMA | 6.5 | 6.5 |
| ORANGE OIL FLORIDA | 6.5 | 6.5 |
| PARA CRESOL METHYL ETHER | 0.3 | 0.3 |
| PATCHOULI RECTIFIED | 3.2 | 3.2 |
| PHENYL ETHYL ACETATE | 0.2 | 0.2 |
| ROSE OXIDE | 0.5 | 0.5 |
| TERPINEOL | 1.6 | 1.6 |
| TERPINYL ACETATE | 5.0 | 5.0 |
| TONALID | 0.3 | 0.3 |
| Total | 100.0 | 100.0 |

The fragrance composition of Example 9 had a strong body floral scent and a strong lilac-like floral feeling, as compared with the fragrance composition of Comparative Example 4.

### Example 10 (Fragrance composition suited for hand cream)

To the fragrance composition base shown in Table 4, the 3-(4-isobutylphenyl)propanenitrile obtained in Example 4 was added so as to attain 10.00 mass%, and the scent/aromatic note was evaluated.

### Comparative Example 5 (Fragrance composition suited for hand cream)

To the fragrance composition base shown in Table 4, 10.00 mass% of dipropylene glycol (DPG) as a solvent was added so as to attain 18.91 mass%, and the scent/aromatic note was evaluated.

**[Table 4]**

| Table 4 | | (mass%) |
|---|---|---|
| | Example 10 | Comparative Example 5 |
| 3-(4-Isobutylphenyl)propanenitrile (Example 4) | 10.00 | - |
| DPG | 8.91 | 18.91 |
| BENZYL ACETATE | 10.00 | 10.00 |
| BORNEOL LAEVO CRIST | 0.18 | 0.18 |
| CINNAMIC ALCOHOL | 2.40 | 2.40 |
| CITRAL SYNTHETIC | 0.50 | 0.50 |
| CITRONELLOL | 5.00 | 5.00 |
| CITRONELLYL ACETATE | 0.18 | 0.18 |
| CITRONELLYL FORMATE | 0.08 | 0.08 |
| CUMINYL ALCOHOL | 0.40 | 0.40 |
| D.M.B.C. ACETATE | 0.07 | 0.07 |
| D.M.P.E. FORMATE | 0.08 | 0.08 |
| EUCALYPTOL | 0.18 | 0.18 |
| EUGENOL | 0.07 | 0.07 |
| GERANIOL | 18.00 | 18.00 |
| GERANYL ACETATE | 0.50 | 0.50 |
| HELIOTROPINE | 7.00 | 7.00 |
| INDOLE PURE | 0.07 | 0.07 |
| IONONE ALPHA | 6.00 | 6.00 |
| LINALOOL | 6.00 | 6.00 |
| LINALYL ACETATE | 11.00 | 11.00 |
| ORANGE TERPENES | 6.00 | 6.00 |
| PARA CRESOL METHYL ETHER | 0.10 | 0.10 |
| PHENYL ACETALDEHYDE | 0.06 | 0.06 |
| PHENYL ETHYL ALCOHOL | 6.50 | 6.50 |
| PHENYL PROPYL ALCOHOL | 0.30 | 0.30 |
| VANILLIN | 0.30 | 0.30 |
| VIRIDINE | 0.12 | 0.12 |
| Total | 100.00 | 100.00 |

The fragrance composition of Example 10 had a strong floral scent and a herbal-rosy geranium-like scent, as compared with the fragrance composition of Comparative Example 5.

### Example 11 (Rose-like fragrance composition)

To the fragrance composition base shown in Table 5, the 3-(4-ethylphenyl)propanenitrile obtained in Example 1 was added so as to attain 7.5 mass%, and the scent/aromatic note was evaluated.

### Comparative Example 6 (Rose-like fragrance composition)

To the fragrance composition base shown in Table 5, dipropylene glycol (DPG) as a solvent was added so as to attain 7.5 mass%, and the scent/aromatic note was evaluated.

**[Table 5]**

| Table 5 | | (mass%) |
|---|---|---|
| | Example 11 | Comparative Example 6 |
| 3-(4-Ethylphenyl)propanenitrile (Example 1) | 7.5 | - |
| DPG | - | 7.5 |
| AMBERONE | 16.0 | 16.0 |
| AMYL SALICYLATE | 2.5 | 2.5 |
| ANISALDEHYDE | 3.6 | 3.6 |
| BACDANOL | 1.0 | 1.0 |
| BENZYL ACETATE | 2.0 | 2.0 |
| CITRONELLOL | 3.0 | 3.0 |
| DIHYDRO MYRCENOL | 1.2 | 1.2 |
| DIMETHYL OCTANOL | 0.4 | 0.4 |
| GERANIOL | 2.6 | 2.6 |
| GERANYL ACETATE | 0.2 | 0.2 |
| HEXYL CINNAMIC ALDEHYDE | 10.0 | 10.0 |
| IONONE ALPHA | 1.8 | 1.8 |
| IONONE BETA | 0.8 | 0.8 |
| KOAVONE | 1.5 | 1.5 |
| LINALOOL | 2.5 | 2.5 |
| LINALYL ACETATE | 0.6 | 0.6 |
| LYSMERAL | 10.0 | 10.0 |
| METHYL ANTHRANILATE | 2.0 | 2.0 |
| METHYL CEDRYL KETONE | 1.0 | 1.0 |
| METHYL IONONE GAMMA | 16.0 | 16.0 |
| ORANGER CRYSTALS | 3.0 | 3.0 |
| PHENOXANOL | 1.5 | 1.5 |
| PHENYL ETHYL ALCOHOL | 2.5 | 2.5 |
| PHENYL ETHYL PHENYL ACETATE | 4.6 | 4.6 |
| ROSE OXIDE | 0.1 | 0.1 |
| VANILLIN | 0.5 | 0.5 |
| YARA YARA | 1.6 | 1.6 |
| Total | 100.0 | 100.0 |

The fragrance composition of Example 11 had a strong floral scent and had a floral scent also in the lasting scent, as compared with the fragrance composition of Comparative Example 6.

As can be understood from the above-described results, it is found that the fragrance composition of the present invention has a strong floral scent. From this, it is found that 3-(4-alkylphenyl)propanenitrile can impart a floral scent, and a green scent or a fruity scent, to the fragrance composition and can further impart the intensity and diffusibility of the scent, and is useful as an active ingredient of the fragrance composition.

In particular, a fragrance composition containing 3-(4-isobutylphenyl)propanenitrile or 3-(4-isopropylphenyl)propanenitrile is more preferable because it has a lilac-like or geranium-like floral feeling.

In addition, a fragrance composition containing 3-(4-n-butylphenyl)propanenitrile is even more preferable because it has a floral and fruity scent and is also excellent in intensity and diffusibility of the scent.

## Claims

1. A fragrance composition, comprising 3-(4-alkylphenyl)propanenitrile represented by General Formula (1) as an active ingredient: 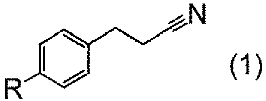 where in Formula (1), R represents an alkyl group having from 2 to 4 carbon atoms.

2. The fragrance composition according to claim 1, further comprising a fragrance substance other than the 3-(4-alkylphenyl)propanenitrile represented by Formula (1).

3. The fragrance composition according to claim 2, wherein the fragrance substance other than the 3-(4-alkylphenyl)propanenitrile represented by Formula (1) comprises at least one selected from the group consisting of hydrocarbons, alcohols, esters, phenols, aldehydes, ketones, acetals, ketals, ethers, natural essential oils, natural extracts, and nitriles other than the 3-(4-alkylphenyl)propanenitrile represented by Formula (1).

4. The fragrance composition according to any one of claims 1 to 3, wherein R is at least one selected from the group consisting of an ethyl group, an isopropyl group, an n-butyl group, an isobutyl group, and a tert-butyl group.

5. The fragrance composition according to any one of claims 1 to 4, wherein R is at least one selected from the group consisting of an isopropyl group, an n-butyl group, and an isobutyl group.

6. The fragrance composition according to any one of claims 1 to 5, wherein R is an n-butyl group.

7. The fragrance composition according to any one of claims 1 to 6, wherein 3-(2-alkylphenyl)propanenitrile represented by General Formula (2) is contained in an amount of from 0.03 to 0.8 mass% based on the 3-(4-alkylphenyl)propanenitrile represented by Formula (1): 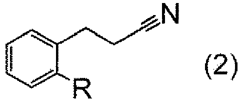 where in Formula (2), R represents an alkyl group having from 2 to 4 carbon atoms.

8. Use of 3-(4-alkylphenyl)propanenitrile represented by General Formula (1) as a fragrance: 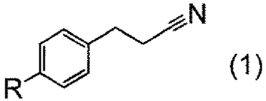 where in Formula (1), R represents an alkyl group having from 2 to 4 carbon atoms.

9. The use according to claim 8, wherein R is at least one selected from the group consisting of an ethyl group, an isopropyl group, an n-butyl group, an isobutyl group, and a tert-butyl group.

10. The use according to claim 8 or 9, wherein the 3-(4-alkylphenyl)propanenitrile represented by Formula (1) imparts a floral scent, and a green scent or a fruity scent.

11. The use according to any one of claims 8 to 10, wherein R is at least one selected from the group consisting of an isopropyl group and an isobutyl group.

12. The use according to claim 11, wherein the 3-(4-alkylphenyl)propanenitrile represented by Formula (1) imparts a floral scent, a green scent, and a fruity scent.

13. The use according to any one of claims 8 to 10, wherein R is an n-butyl group.

14. The use according to claim 13, wherein the 3-(4-alkylphenyl)propanenitrile represented by Formula (1) imparts a floral scent, a fruity scent, and a spicy scent.

15. A scenting method, comprising imparting a floral scent, and a green scent or a fruity scent, with 3-(4-alkylphenyl)propanenitrile represented by General Formula (1): 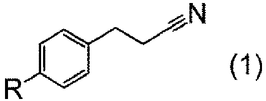 where in Formula (1), R represents an alkyl group having from 2 to 4 carbon atoms.

16. A method for producing 3-(4-alkylphenyl)propanenitrile, the method comprising, in this order: a step of converting, via a condensation reaction, 4-alkylbenzaldehyde represented by Formula (3) as a raw material and acetonitrile into cinnamonitrile represented by Formula (4); and a hydrogenation step: 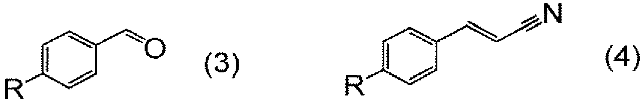 where in Formulae (3) and (4), R represents an alkyl group having from 2 to 4 carbon atoms.

17. A nitrile composition, comprising 3-(4-alkylphenyl)propanenitrile represented by General Formula (1) and 3-(2-alkylphenyl)propanenitrile represented by General Formula (2), wherein the 3-(2-alkylphenyl)propanenitrile represented by General Formula (2) is contained in an amount of from 0.03 to 0.8 mass% based on the 3-(4-alkylphenyl)propanenitrile represented by Formula (1): 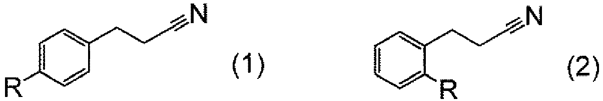 where in Formula (1), R represents an alkyl group having from 2 to 4 carbon atoms, and, in Formula (2), R represents an alkyl group having from 2 to 4 carbon atoms.
